# EUROPEAN PATENT APPLICATION

(11) **EP 3 340 093 A1**
(43) Date of publication of application: **27.06.2018**
(21) Application number: 17182771.0
(22) Date of filing: 24.07.2017
(51) Int. Cl.: G06F 19/00, G06Q 50/22, A61B 6/00, G16H 40/20

(54) **METHOD FOR DETECTING AND ANALYSING A WORKFLOW PERFORMED WITH AN IMAGING MODALITY**

(71) Applicant: Siemens Healthcare GmbH, 91052 Erlangen (DE)
(72) Inventor: Ferguson, George William, 91056 Erlangen (DE); Nufer, Stephan, 91056 Erlangen (DE)

(57) **Abstract**

The present invention relates to a method for detecting and analysing a workflow consisting of work steps performed in an examination room of an imaging modality, in particular during a patient examination, the method comprises the following steps:
- recording first detection data of a first position on or close to a patient table in the examination room and/or recording second detection data of a second position anywhere else in the examination room by means of a detection unit;
- wherein the first and second detection data are continuously recorded as a function of time;
- analysing the detection data by means of an analysing unit, wherein the analysis of the detection data comprises:
• identifying whether at least one action has taken place in the examination room,
• recording the time and/or the time interval in which the at least one action has taken place, and
• identifying the type of the at least one action which has taken place, and recording the type of the at least one action.

Furthermore, the present invention relates to an imaging modality, a computer program and an electronically readable data carrier configured to perform the proposed method.

## Description

The present invention relates to a method for detecting and analysing a workflow performed with an imaging modality. Thus, the present invention relates to the technical field of performing an examination of a patient with an imaging modality, for example, a computer tomography modality, a magnetic resonance modality, or the like.

Nowadays, to gather statistics about a duration of an examination, single work steps performed during an examination are recorded manually using a stopwatch. However, recording single work steps during an examination is time-consuming and inaccurate. As a result, the analysis of the workflow consisting of work steps performed is inexact, since only the start and the end of an acquisition can be detected accurately. Last but not least, no exact data can be acquired about the time intervals in which an imaging modality is not used.

Therefore, the usage of an imaging modality is usually not optimized.

For example, DE 10 2015 211 148 A1 discloses the detection of positioning data of a patient when the patient is placed on a patient table by means of a positioning detection unit. The position data detected reproduce a surface image of the patient, i.e., the anatomy of the patient.

In order to optimize the usage of an imaging modality, however, information about the image acquisition time needed for performing an examination of a patient and information about the time or time intervals for performing work steps in preparation and/or follow-up work steps are needed.

Therefore, an objective technical task of the present invention is to provide a method for improving an utilisation rate of an imaging modality and to provide an imaging modality that can be used effectively.

This task is solved by a method for detecting and analysing a workflow consisting of work steps performed in an examination room of an imaging modality according to claim 1 and by an imaging modality according to claim 11. Furthermore, the above task is solved by a computer program according to claim 15 and an electronically readable data carrier according to claim 16.

According to the invention, a method for detecting and analysing a workflow consisting of work steps performed in an examination room of an imaging modality, in particular, during a patient examination, is proposed. The method comprises the following steps:
- recording first detection data of a first position on or close to a patient table in the examination room and/or recording second detection data of at least a second position anywhere else in the examination room by means of at least one detection unit;
- wherein the first and second detection data are continuously recorded as a function of time;
- analysing the detection data by means of an analysis unit, wherein the analysis of the detection data comprises:
   - identifying whether at least one action has taken place in the examination room,
   - recording the time and/or the time interval in which the at least one action has taken place, and
   - identifying the type of the at least one action which has taken place, and recording the type of the at least one action.

According to the proposed method, the work steps of preparing an examination, performing the same, and processing follow-up work steps are recorded by the detection unit. Since the different work steps are recorded continuously as a function of time, the different work steps are recorded accurately. This allows to analyse in detail the workflow of an imaging modality, for example, a computer tomography modality, a magnetic resonance modality, or the like. Since a first position is detected which detects whether an action has taken place in the area of the patient table, and since at least a second position anywhere else in the examination room is detected which detects whether an action has taken place in another area of the examination room, the detected data can be recorded accurately and completely, allowing an reliable analysis of the usage of the imaging modality, i.e., the utilization rate of the imaging modality. The actions in the other area can be, for example, detecting whether a coil needed for the examination is placed, monitoring access to the examination room, or the like.

According to an embodiment of the invention, the step of identifying whether at least one action has taken place in the examination room is based on a movement detection. The movement detection could be performed using a camera and/or a movement sensor. The movement detection allows for continuous and accurate detection of whether an action has taken place, e.g. whether a patient or an operator has moved or has been moved in some way, and detection of how much time was needed for performing said action.

According to a further embodiment of the invention, the step of identifying the type of the at least one action is based on a detection data analysis of the detection data recorded by the detection unit, wherein the detection unit comprises at least one camera, in particular, a 2D or 3D camera, and/or at least one movement sensor. By detecting the detection data with a camera and/or a movement sensor, the evaluation of the detection data can be performed reliably since two different types of data can be detected and evaluated. For example, a video recorded by the camera needs only be evaluated to identify the type of action, during the times that movement is detected by the movement sensor. The two different types of detection data can be compared automatically so that errors in evaluating the detected data can be prevented. Preferably, the camera used is an optical camera. The optical camera takes images in series, which when evaluated provide information, whether a particular action has taken place. For example, a particular action may be identified when a specific pattern is recognized in the detection data. It is possible for example, that the operator or user wears clothes of a particular colour that is recognized easily by the detection unit or camera respectively. In such a case, the actions performed by the operator have to be actions belonging to the categories "patient preparation" or "patient release".

According to a further embodiment of the invention, the step of identifying the type of the at least one action is based on a Deep Learning algorithm. The Deep Learning algorithm belongs to a class of optimization methods using artificial neural networks. The advantage of using such a Deep Learning algorithm is that by repeatedly detecting the same type of actions, the identifying of said action becomes more reliable each time it is detected.

According to a further embodiment of the invention, the at least one action is one of the following actions:
- a patient enters the examination room,
- a patient is placed on the patient table,
- a device used for performing the examination, in particular a magnetic resonance coil, ear protection, and/or an emergency button, are placed on the patient,
- establishing a vascular access to administer a medicine, in particular a contrast agent,
- the patient is moved into an iso-centre of the imaging modality,
- the imaging modality is acquiring navigator images,
- the imaging modality is acquiring diagnostic images
- the patient is moved out of the iso-centre,
- the device used for performing the examination, in particular the magnetic resonance coil, ear protection and/or emergency button, are removed from the patient,
- the patient gets up from the patient table,
- the patient leaves the examination room,
- maintenance staff for performing maintenance work is in the examination room and/or
- nobody is present in the examination room.

The different types of actions that are detected belong to different types of categories. This means that the different types of actions can be aggregated to different categories, such as "patient registration", "patient preparation", "image acquisition" etc.. The analysis can thus be focused on these categories that show which type of actions require most of the time and which type of actions require the least time of an examination. Such accurate analysis allows for an optimized utilization of the imaging modality, in particular it can allow for a parallelization of the work steps that are needed to be performed.
According to a further embodiment of the invention, the data entered into and/or recorded by the imaging modality, in particular acquisition times and/or registration data and/or registration times of a patient, are considered in order to perform a reliable analysis. This means different types of actions are detected by the detection unit and that the data acquired during the examination as such are analysed and used to perform a proper analysis of the entire examination, in particular as a function of time. This has the particular advantage that a complete analysis can be performed automatically.

According to a further embodiment of the invention, the method comprises the additional step of classifying the at least one identified action into different categories, wherein the different categories refer to generic terms such as registration, acquisition time, adjustment time, or maintenance time. The term registration comprises the registration of the patient by entering the name of the patient and/or the weight and/or the age and/or the already known anamnesis of the patient into a control unit of the imaging modality. The image acquisition time comprises the time intervals for performing the medical examination as such. The adjustment time comprises the time intervals for preparing the patient for the examination, for example, by placing coils, ear protection, and the like on or close to the patient. The maintenance time comprises the time intervals for performing maintenance work on the imaging modality. By assigning the different types of detected actions to different categories, the analysis of utilisation of the imaging modality can be performed accurately and in detail.

According to a further embodiment of the invention, the sequence of identified actions in one patient examination is analysed with regard to the corresponding recorded times and time intervals of each identified action, and, in particular, the categories of the identified actions. Therewith, the time needed for each patient can be estimated reliably. This has the advantage that the daily utilisation of the imaging modality can be planned such that the dead time of the imaging modality can be minimized.

According to a further embodiment of the invention, the analysis unit analyses a number of patient examinations and calculates an average time interval for each category of identified actions for several imaging modalities, wherein the average time intervals are compared with one another. By analysing a number of patient examinations, the average time interval for each category can be calculated. This allows for analysing which action or which type of action requires which amount of time on average.

According to a further embodiment of the invention, the recorded times and/or time intervals of one imaging modality or several imaging modalities are shown on a monitor to a user. This has the advantage that the user can effectively plan the utilisation of the different modalities so that dead times of a modality can be prevented.

According to the proposed method, the different types of actions detected as a function of time can be assigned to different categories, which in turn allows for a detailed and reliable analysis of the utilization of the imaging modality, in particular in regard of dead times of the imaging modality or the like. Such a reliable analysis may be used to optimize the time schedule of the different work steps in order to optimize the utilization of the imaging modality. Furthermore, the detected data of a single modality as well as of a plurality of image modalities established in a facility can be detected and compared with one another. Last but not least detected data of several facilities may be compared with one another. In this latter case the facilities can be connected to a cloud based network, for example like "Teamplay".

The proposed method offers an effective tool to obtain a statistically reliable analysis. For example particular imaging modalities may be identified that work efficiently and other imaging modalities may be identified that work less efficiently. Such an analysis in turn allows for qualitative investigations of the factors why a particular imaging modality works efficiently or less efficiently. Said factor can be improved or prevented in order to optimize the utilization of an imaging modality.

Furthermore, it is possible to provide the detected data in a clear format to a client operating several imaging modalities. The detected data can be supplied via "Teamplay" to the client. In this way the client may realize which imaging modality is operated efficiently and which one is operated less efficiently. The client is thus given the possibility to improve the efficiency of its imaging modalities.

According to another aspect of the present invention, an imaging modality is proposed, wherein the imaging modality comprises:
a first detection unit for detecting first detection data of a first position of a patient table in the examination room and/or a second detection unit for detecting second detection data of a second position anywhere else in the examination room,
an analysis unit for analysing the detection data, wherein the first and the second detection unit communicates the detected data to the analysis unit, and
a control unit for controlling the detection unit and/or the analysis unit, wherein the imaging modality is configured to perform a method as described herein.

According to the proposed imaging modality, the work steps of preparing an examination, performing the same, and processing follow-up work steps are recorded close to the patient table and/or close to a position anywhere in the examination room by the detection unit. Since the different work steps are recorded continuously as a function of time, the different work steps are recorded accurately. This allows an analysis unit to analyse in detail the workflow of an imaging modality, such as for example a computer tomography modality, a magnetic resonance modality, or the like. The control unit is provided for controlling the detection unit and/or the analysis unit so that, for example, the control unit controls that the detection unit is supplying the analysing unit with the detected data. Furthermore, the control unit can be configured to supply the analysis unit with data detected by the imaging modality during an examination. Since a first position is detected which detects whether an action has taken place in the area of the patient table, and since at least a second position anywhere else in the examination room is detected which detects whether an action has taken place in another area of the examination room, the detected data can be recorded accurately and completely, allowing an reliable analysis of the usage of the imaging modality. The actions in the other area can be, for example, a detecting whether a coil needed for the examination is placed, monitoring an access to the examination room, or the like.

According to a further embodiment of the proposed imaging modality, the detection unit comprises at least one camera, in particular a 3D camera, for recording optical images and/or at least one movement sensor for recording the at least one action, in particular a movement. Further examples of a movement sensor are given for example by a breathing sensor for monitoring the breathing of a patient, or by a sensor for monitoring an electrocardiogram for monitoring the cardiac activity of a patient. The detected data detected by the detection unit can be analysed by using, for example, a Deep Learning algorithm. This means that the analysis can be performed automatically.

According to a further embodiment of the proposed imaging modality, the detection data of the first and the second detection unit are analysed individually. After analysing the detected data of the first and the second detection unit individually, the detection data are assigned to a particular category. In this way the detection data of one category that are acquired in different examinations can be compared with one another and/or are averaged.

According to a further embodiment of the proposed imaging modality, the analysis unit is supplied with data entered into and/or recorded by the imaging modality for performing a reliable analysis. The data entered into the imaging modality comprises the data that are aggregated under the category registration, for example the name, the weight, the age and/or the anamnesis of a patient. The data recorded by the imaging modality comprises the data detected during an examination of the patient. The data recorded by the imaging modality can be magnetic resonance data, chromatography data, or the like. The data recorded comprises in particular times and durations of image acquisitions, etc.

According to a further embodiment of the proposed imaging modality, the second position is at or close to the access to the imaging modality and/or the connection area of a magnetic resonance coil used for the examination. By detecting the second position, all actions or most of the actions taking place besides the patient table can be detected and can be analysed. For example the coils used for a particular examination are usually plugged into the respective socket during patient preparation. Therefore the plugging and the unplugging of the corresponding coils are detectable as at least one action that is detected in the first or the second position. Therewith, the analysis of the utilisation of an imaging modality can be improved.

According to another aspect of the present invention, a computer program is proposed that implements a method as described in a control unit of an imaging modality when the computer program is executed on the control unit.

According to another aspect of the present invention, an electronically readable data carrier with electronically readable control information stored thereon is proposed, which at least comprises a computer program previously described and which is configured such that, when the data carrier is used in a control device of an imaging modality a method described herein is performed.

Further advantages, features and characteristics of the present invention result from the following description of advantageous embodiments of the invention.

The figures show
- Fig. 1: a magnetic resonance modality,
- Fig. 2: a flow chart of the proposed method, and
- Fig. 3: an example of a time schedule for the utilization of the detection unit and the imaging modality during an examination of a patient.

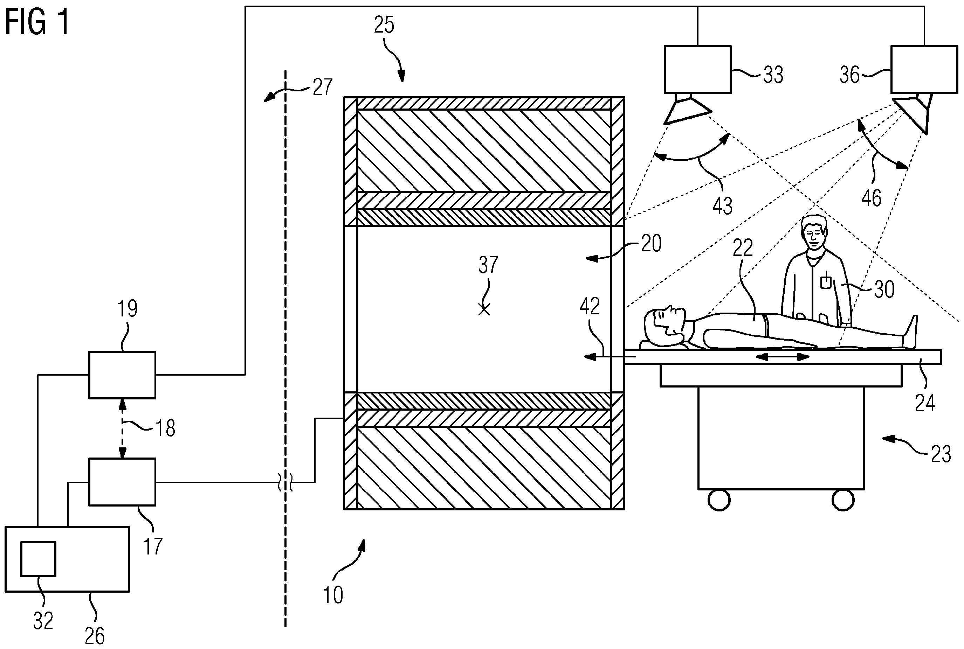

The Figure 1 shows an imaging modality 10, for example, a magnetic resonance modality, a tomography modality, or the like. The imaging modality 10 shown in Figure 1 comprises an iso-centre 37 into which a patient table 24 of a patient support 23 is movable. A patient table 24 of the patient support 23 on which a patient 22 is placed is movable along a direction 42 into the iso-centre 37 of the imaging modality 10. The iso-centre 37 is located in an acquisition area 20 of the imaging modality 10. A user 30, in particular a physician, places a device used during the examination, for example, a magnetic resonance coil or ear protection, on the patient 22 before the patient is moved into the acquisition area 20.
The imaging modality 10 and the patient table 24 are located in an examination room 25. The examination room 25 is recorded by a first detection unit 33 and/or a second detection unit 36. The first detection unit 33 is positioned such that it detects the actions performed close to the patient table 24. The second detection unit 36 detects a second position anywhere else in the examination room 25, in particular, an access to the examination room or an area for connecting an examination coil needed for the examination or the like.

The first detection unit 33 may be pivotable along an angle area 43 and the second detection unit 36 may be pivotable along an angle area 46. The first detection unit 33 and the second detection unit 36 each comprises a camera and/or a movement sensor for detecting whether an action has taken place. The first detection unit 33 and the second detection unit 36 are electronically connected to a control unit 19. The control unit 19 is connected to an analysis unit 26, so that the control unit 19 can supply the received detection data from the first and/or the second detection unit 33, 36 to the analysis unit 26. The analysis unit 26 is connected to an acquisition unit 17 or the imaging modality 10, which acquires the image acquisition data detected during an examination. The imaging modality 10 supplies the detected image acquisition data to the analysing unit 26. The control unit 19 is connected to the acquisition unit 17 via the connection 18. The control unit 19 can control the acquisition unit 17 such that it allows the acquisition unit 17 to supply the acquisition data to the analysis unit 26 only when the first detection unit 33 detects that the patient table 24 is positioned, in particular, mainly within the acquisition area 20. The analysis unit 26 is provided with a monitor 32 onto which the analysis of the detection data can be shown to a user 30 and which is positioned in a control room 27 being adjacent to the examination room 25.

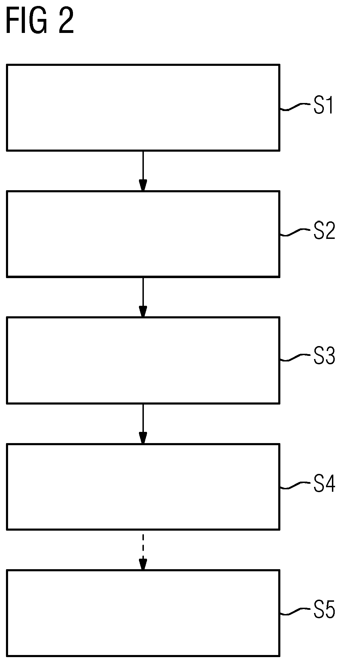

Figure 2 shows a flow chart of the proposed method. In a first step S1 a first and/or a second detection data is recorded in, respectively, a first or second position in the examination room 25. In step S1, the first and the second detection data are recorded continuously as a function of time.

In a second step S2, the detection data are analysed by means of an analysis unit 26. In the third step S3, it is identified whether at least one action has taken place. If an action has taken place, the time and/or the time interval in which the at least one action has taken place is evaluated. In the fourth step S4, the type of the at least one action that has taken place is identified and recorded. The identification of an action that has taken place and/or the type of action is analysed using a Deep Learning algorithm.

In a fifth step S5, the analysing unit 26 is supplied with data, in particular, acquisition data that has been entered and/or recorded by the acquisition unit 17 of the imaging modality 10.

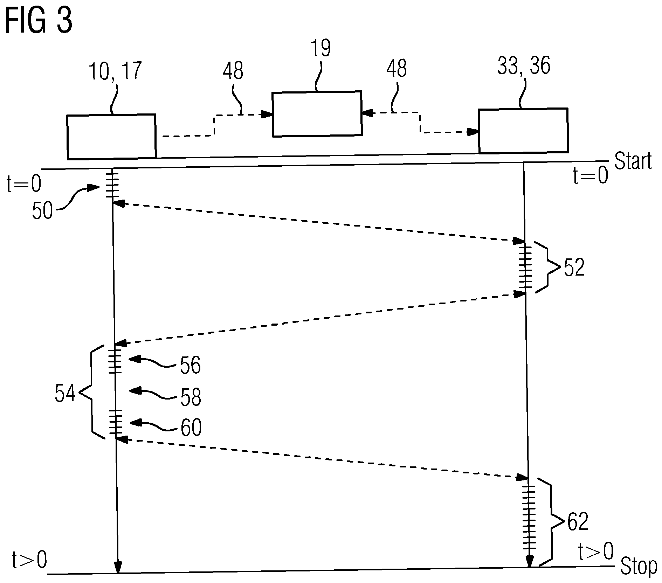

Figure 3 shows an example of a time schedule of the utilization of the detection unit and the imaging modality during one examination of a patient 22. An examination starts at a time t=0 when a user 30 or an operator respectively starts entering patient information into the imaging modality 10. This step corresponds to the category "registration 50". The same examination stops at a time t>0, when the patient leaves the examination room 25.

The registration 50 comprises for example typing in the patient's name, the patient's age, the patient's sex, the patient's weight, the patient's height, or the like into the imaging modality 10. As long as the operator or user 30 enters the patient information during the registration 50 into the imaging modality 10, the detection units 33, 36 are expected to detect no action. Thus the detection units 33, 36 are in a "stand-by mode" corresponding to a detection of a dead time interval. The patient information are typed into the imaging modality 10 and are automatically supplied to the control unit 19 through a connection 48.

After the registration 50 is performed the patient 22 is prepared for the examination. The actions performed for the "patient preparation 52" are detected by the detection units 33, 36 and may comprise for example the actions "the patient enters the examination room 25", "placing the patient 22 on the patient table 24", "placing a magnetic coil used for the examination on the patient 22" or "plugging said coil into a predetermined position on the patient table 24", "establishing a vascular access to administer a medicine", or the like. During the step of patient preparation 52 the detection units 33, 36 are in an active mode, while the imaging modality 10 is preferably in an inactive mode, i.e. a stand-by mode. The inactive mode corresponds to a state, in which the imaging modality 10 acquires or receives no data. The detection data detected by the detection units 33, 36 are automatically supplied to the control unit 19 through the connection 48. The supply of data from the imaging modality 10 or from the detection units 33, 36 are indicated by reference sign 48. After the step "patient preparation 52" is performed a step of "image acquisition 54" follows, in which the imaging modality 10 is in an active mode. During the step of image acquisition 56 the following actions may be detected by the imaging modality 10. At the beginning of the step of image acquisition 54, navigator images may be detected in a sub-step 56. After the navigator images are detected in sub-step 56, these navigator images may be evaluated by the operator in a further sub-step 58. During evaluation of the navigator images in sub-step 58, the imaging modality is in an inactive mode, i.e. a dead time of the imaging modality is detected. After the evaluation of the navigator images in sub-step 58 a further sub-step 60 may follow, in which diagnostic images are acquired. The acquisition of the diagnostic images correspond to the proper examination of the patient 22. The substeps 56 and 60 are comprised in the category of "image acquisition 54". The step 58 may be comprised in a category "dead time of the imaging modality". While the step 54 is performed, the detection units 33, 36 usually detect no action. Thus the detection units 33, 36 are in an inactive mode, like a stand-by mode.

After the image acquisition 54 is finished the patient 22 is removed from the imaging modality 10, by moving out the patient table 24 from the imaging modality 10. In a step of a "patient release 62" the detections units 33, 36 are again in an active mode for detecting actions like, for example, releasing the patient 22 from the vascular access or the like, the patient 22 gets up from the patient table 24, the patient 22 leaves the examination room 25. After the patient 22 has left the examination room 25 the examination is completed.

In summary, this means a particular examination starts with step 50, when the patient is registered and stops when the patient leave the examination room 25 in step 62. When the steps 50 to 62 are performed either the imaging modality 10 is active, while the detection units 33, 36 are usually inactive, or the detection units 33, 36 are active, while the imaging modality 10 is usually inactive.

However, it is possible to perform the steps 50 and 52 in parallel. In this case a first operator may enter the patient information, and a second operator may prepare the patient. Since however the detected data are supplied automatically to the control unit 19 as a function of time, all the detected actions may be graded as a function of time and as a function of a particular category.

The proposed method for detecting and analysing the workflow performed with an imaging modality provides a tool with which the utilization of the imaging modality can be analysed and improved.

## Claims

1. Method for detecting and analysing a workflow consisting of work steps (S1 - S4) performed in an examination room (25) of an imaging modality (10), in particular during a patient examination, the method comprises the following steps:
- recording first detection data of a first position on or close to a patient table (24) in the examination room (25) and/or recording second detection data of a second position anywhere else in the examination room (25) by means of a detection unit (33, 36);
- wherein the first and second detection data are continuously recorded as a function of time;
- analysing the detection data by means of an analysing unit (26), wherein the analysis of the detection data comprises:
• identifying whether at least one action has taken place in the examination room (25),
• recording the time and/or the time interval in which the at least one action has taken place, and
• identifying the type of the at least one action which has taken place, and recording the type of the at least one action.

2. Method according to claim 1, wherein the step of identifying whether at least one action has taken place in the examination room (25) is based on a movement detection.

3. Method according to claim 1 or 2, wherein identifying the type of the at least one action is based on a detection data analysis of the detection data recorded by the detection unit (33, 36), wherein the detection unit (33, 36) comprises at least one camera, in particular a 2D or 3D camera, and/or at least one movement sensor.

4. Method according to one of the preceding claims, wherein identifying the type of the at least one action is based on a Deep Learning algorithm.

5. Method according to one of the preceding claims, **characterized in that** the at least one action is one of the following actions:
- a patient (22) enters the examination room (25),
- a patient (22) is placed on the patient table (24),
- a device used for performing the examination, in particular a magnetic resonance coil, a hearing protection, and/or an emergency button, are placed on the patient (22),
- establishing a vascular access to administer a medicine, in particular a contrast agent,
- the patient (22) is moved into an iso-centre (37) of the imaging modality (10),
- the imaging modality (10) is acquiring navigator images,
- the imaging modality (10) is acquiring diagnostic images
- the patient (22) is moved out of the iso-centre (37),
- the device used for performing the examination, in particular the magnetic resonance coil, the hearing protection and/or the emergency button, are removed from the patient (22),
- the patient (22) gets up from the patient table (24),
- the patient (22) leaves the examination room (25),
- maintenance staff for performing maintenance work is in the examination room (25) and/or
- nobody is present in the examination room (25).

6. Method according to one of the preceding claims, **characterized in that** data entered into and/or recorded by the imaging modality (10), in particular image acquisition times and/or registration data and/or registration times of a patient (22), are considered in the analysis of the detection data.

7. Method according to one of the preceding claims, comprising the additional step of classifying the at least one identified action into different categories, wherein the different categories refer to generic terms such as registration, acquisition time, adjustment time or maintenance time.

8. Method according to one of the preceding claims, wherein the sequence of identified actions in a patient examination is analysed with regard to the corresponding recorded times and time intervals of each identified action, and in particular the categories of the identified actions.

9. Method according to one of the preceding claims, **characterized in that** the analysing unit (26) analyses a number of patient examinations and calculates an average time interval for each category of identified actions for several imaging modalities, wherein the average time intervals are compared with one another.

10. Method according to one of the preceding claims, **characterized in that** the recorded times and/or time intervals of one imaging modality (10) or several imaging modalities (10) are shown on a monitor (32) to a user (30).

11. An imaging modality comprising
a first detection unit (33) for detecting first detection data of a first position of a patient table (24) in the examination room (25) and/or a second detection unit (36) for detecting second detection data of a second position anywhere else in the examination room (25),
an analysis unit (26) for analysing the detection data, wherein the first and the second detection unit (33, 36) communicates the detected data to the analysing unit (26), and
a control unit (19) for controlling the detection unit (33, 36) and/or the analysing unit (26), wherein the imaging modality (10) is configured to perform the method according to any one of the preceding claims.

12. Imaging modality (10) according to claim 11, **characterized in that** the detection unit (33, 36) comprises at least one camera, in particular a 2D or 3D camera, for recording optical images and/or at least one movement sensor for recording the at least one action, in particular a movement.

13. Imaging modality (10) according to one of the claims 11 to 12, **characterized in that** the analysing unit (26) is supplied with data entered and/or recorded by the imaging modality (10) for performing a reliable analysis.

14. Imaging modality (10) according to one of the claims 11 to 13, **characterized in that** the second position is at or close to the access to the imaging modality (10) and/or the connection area of a magnetic resonance coil used for the examination.

15. Computer program implementing a method according to any one of the claims 1 to 10 in a control unit (19) of an imaging modality (10), when the computer program is executed on the control unit (19).

16. An electronically readable data carrier with electronically readable control information stored thereon, which at least comprises a computer program according to claim 16 and is configured such that, when the data carrier is used in a control device (19) of an imaging modality (10), in particular to one of the claims 11 to 15, a method according to one of claims 1-14 is performed.

## Amended claims

### Amended claims in accordance with Rule 137(2) EPC.

1. Method for detecting and analysing a workflow consisting of work steps (S1 - S4) performed in an examination room (25) of an imaging modality (10), in particular during a patient examination, the method comprises the following steps:
- recording first detection data of a first position on or close to a patient table (24) in the examination room (25) and/or recording second detection data of a second position anywhere else in the examination room (25) by means of a detection unit (33, 36);
- wherein the first and second detection data are continuously recorded as a function of time;
- analysing the detection data by means of an analysing unit (26), wherein the analysis of the detection data comprises:
• identifying whether at least one action has taken place in the examination room (25),
• recording the time and/or the time interval in which the at least one action has taken place, and
• identifying the type of the at least one action which has taken place, and recording the type of the at least one action,
wherein data entered into and/or recorded by the imaging modality (10), in particular image acquisition times and/or registration data and/or registration times of a patient (22), are considered in the analysis of the detection data.

2. Method according to claim 1, wherein the step of identifying whether at least one action has taken place in the examination room (25) is based on a movement detection.

3. Method according to claim 1 or 2, wherein identifying the type of the at least one action is based on a detection data analysis of the detection data recorded by the detection unit (33, 36), wherein the detection unit (33, 36) comprises at least one camera, in particular a 2D or 3D camera, and/or at least one movement sensor.

4. Method according to one of the preceding claims, wherein identifying the type of the at least one action is based on a Deep Learning algorithm.

5. Method according to one of the preceding claims, **characterized in that** the at least one action is one of the following actions:
- a patient (22) enters the examination room (25),
- a patient (22) is placed on the patient table (24),
- a device used for performing the examination, in particular a magnetic resonance coil, a hearing protection, and/or an emergency button, are placed on the patient (22),
- establishing a vascular access to administer a medicine, in particular a contrast agent,
- the patient (22) is moved into an iso-centre (37) of the imaging modality (10),
- the imaging modality (10) is acquiring navigator images,
- the imaging modality (10) is acquiring diagnostic images
- the patient (22) is moved out of the iso-centre (37),
- the device used for performing the examination, in particular the magnetic resonance coil, the hearing protection and/or the emergency button, are removed from the patient (22),
- the patient (22) gets up from the patient table (24),
- the patient (22) leaves the examination room (25),
- maintenance staff for performing maintenance work is in the examination room (25) and/or
- nobody is present in the examination room (25).

6. Method according to one of the preceding claims, comprising the additional step of classifying the at least one identified action into different categories, wherein the different categories refer to generic terms such as registration, acquisition time, adjustment time or maintenance time.

7. Method according to one of the preceding claims, wherein the sequence of identified actions in a patient examination is analysed with regard to the corresponding recorded times and time intervals of each identified action, and in particular the categories of the identified actions.

8. Method according to one of the preceding claims, **characterized in that** the analysing unit (26) analyses a number of patient examinations and calculates an average time interval for each category of identified actions for several imaging modalities, wherein the average time intervals are compared with one another.

9. Method according to one of the preceding claims, **characterized in that** the recorded times and/or time intervals of one imaging modality (10) or several imaging modalities (10) are shown on a monitor (32) to a user (30).

10. An imaging modality comprising
a first detection unit (33) for detecting first detection data of a first position of a patient table (24) in the examination room (25) and/or a second detection unit (36) for detecting second detection data of a second position anywhere else in the examination room (25),
an analysis unit (26) for analysing the detection data, wherein the first and the second detection unit (33, 36) communicates the detected data to the analysing unit (26), and
a control unit (19) for controlling the detection unit (33, 36) and/or the analysing unit (26), wherein the imaging modality (10) is configured to perform the method according to any one of the preceding claims.

11. Imaging modality (10) according to claim 10, **characterized in that** the detection unit (33, 36) comprises at least one camera, in particular a 2D or 3D camera, for recording optical images and/or at least one movement sensor for recording the at least one action, in particular a movement.

12. Imaging modality (10) according to one of the claims 10 to 11, **characterized in that** the analysing unit (26) is supplied with data entered and/or recorded by the imaging modality (10) for performing a reliable analysis.

13. Imaging modality (10) according to one of the claims 10 to 12, **characterized in that** the second position is at or close to the access to the imaging modality (10) and/or the connection area of a magnetic resonance coil used for the examination.

14. Computer program implementing a method according to any one of the claims 1 to 9 in a control unit (19) of an imaging modality (10), when the computer program is executed on the control unit (19).

15. An electronically readable data carrier with electronically readable control information stored thereon, which at least comprises a computer program according to claim 14 and is configured such that, when the data carrier is used in a control device (19) of an imaging modality (10), in particular to one of the claims 10 to 14, a method according to one of claims 1-13 is performed.
